# EUROPEAN PATENT APPLICATION

(11) **EP 4 230 607 A1**
(43) Date of publication of application: **23.08.2023**
(21) Application number: 21880012.6
(22) Date of filing: 08.10.2021
(51) Int. Cl.: C07C 21/18, F17C 1/10, F17C 13/00, C07C 17/38

(54) **GAS-FILLED CONTAINER AND METHOD OF STORING (E)-1,1,1,4,4,4-HEXAFLUORO-2-BUTENE**

(30) Priority: 15.10.2020 JP 2020173920
(71) Applicant: Resonac Corporation, Tokyo 105-8518 (JP)
(72) Inventor: TANIMOTO Yosuke, Tokyo 105-8518 (JP); SUZUKI Atsushi, Tokyo 105-8518 (JP)
(74) Representative: Strehl Schübel-Hopf & Partner
(86) International application number: PCT/JP2021/037427
(87) International publication number: WO 2022/080273

(57) **Abstract**

There is provided a gas-filled container in which the purity of filled (E)-1,1,1,4,4,4-hexafluoro-2-butene is less likely to decrease over a long term. The gas-filled container includes: a filled container filled with (E)-1,1,1,4,4,4-hexafluoro-2-butene. A part in contact with the filled (E)-1,1,1,4,4,4-hexafluoro-2-butene of the filled container is formed of a metal having a copper concentration of less than 0.5% by mass.

## Description

### Technical Field

The present invention relates to a gas-filled container and a method for storing (E)-1,1,1,4,4,4-hexafluoro-2-butene.

### Background Art

To stably perform fine processing in dry etching, a dry etching gas is demanded to have a high purity (e.g., 99.9% by volume or more). Further, the dry etching gas is stored in a state of being filled in a filled container until use, and therefore the high purity needs to be maintained over a long term in the filled container.

PTL 1 discloses a technology of storing 2-fluorobutane, 2-fluoro-2-methylpropane, and 2-fluoropentane usable as a dry etching gas while maintaining the high purity over a long term. The technology disclosed in PTL 1 suppresses a decrease in purity by filling 2-fluorobutane, 2-fluoro-2-methylpropane, or 2-fluoropentane into a manganese steel container having a small amount of aluminum adhering to the inner surface.

### Citation List

### Patent Literature

PTL 1: WO 2016/117464

### Summary of Invention

### Technical Problem

It has been examined to utilize (E)-1,1,1,4,4,4-hexafluoro-2-butene as a dry etching gas for manufacturing semiconductors. However, (E)-1,1,1,4,4,4-hexafluoro-2-butene has a double bond in the molecule, and thus has stability lower than that of 2-fluorobutane, 2-fluoro-2-methylpropane, and 2-fluoropentane, so that (E)-1,1,1,4,4,4-hexafluoro-2-butene has easily caused reactions, such as isomerization, polymerization, and decomposition, during storage.

Therefore, when the technology disclosed in PTL 1 is applied to (E)-1,1,1,4,4,4-hexafluoro-2-butene, the reactions, such as isomerization, polymerization, and decomposition, have progressed during long-term storage, so that the purity has decreased in some cases.

It is an object of the present invention to provide a gas-filled container in which the purity of the filled (E)-1,1,1,4,4,4-hexafluoro-2-butene is less likely to decrease over a long term and a method for storing (E)-1,1,1,4,4,4-hexafluoro-2-butene.

### Solution to Problem

To solve the above-described problems, one aspect of the present invention is as described in [1] to [13] below.
[1] A gas-filled container includes: a filled container filled with (E)-1,1,1,4,4,4-hexafluoro-2-butene, in which
   a part in contact with the filled (E)-1,1,1,4,4,4-hexafluoro-2-butene of the filled container is formed of a metal having a copper concentration of less than 0.5% by mass.
[2] The gas-filled container according to [1], in which the part in contact with the filled (E)-1,1,1,4,4,4-hexafluoro-2-butene of the filled container is formed of a metal having a copper concentration of less than 0.4% by mass.
[3] The gas-filled container according to [1] or [2], in which the metal is steel.
[4] The gas-filled container according to [1] or [2], in which the metal is at least one of manganese steel and chromium molybdenum steel.
[5] The gas-filled container according to any one of [1] to [4], in which the copper concentration is measured by X-ray photoelectron spectroscopy.
[6] The gas-filled container according to any one of [1] to [5], in which the filled container includes: a cylinder containing the (E)-1,1,1,4,4,4-hexafluoro-2-butene; and a valve configured to open and close a flow passage for causing the (E)-1,1,1,4,4,4-hexafluoro-2-butene inside the cylinder to flow to the outside.
[7] The gas-filled container according to any one of [1] to [6], in which the purity of the (E)-1,1,1,4,4,4-hexafluoro-2-butene to be filled is 99.90% by volume or more.
[8] A method for storing (E)-1,1,1,4,4,4-hexafluoro-2-butene includes: filling (E)-1,1,1,4,4,4-hexafluoro-2-butene into a container for storage, in which
   a part in contact with the filled (E)-1,1,1,4,4,4-hexafluoro-2-butene of the filled container is formed of a metal having a copper concentration of less than 0.5% by mass.
[9] The method for storing (E)-1,1,1,4,4,4-hexafluoro-2-butene according to [8], in which the part in contact with the filled (E)-1,1,1,4,4,4-hexafluoro-2-butene of the filled container is formed of a metal having a copper concentration of less than 0.4% by mass.
[10] The method for storing (E)-1,1,1,4,4,4-hexafluoro-2-butene according to [8] or [9], in which the metal is steel.
[11] The method for storing (E)-1,1,1,4,4,4-hexafluoro-2-butene according to [8] or [9], in which the metal is at least one of manganese steel and chromium molybdenum steel.
[12] The method for storing (E)-1,1,1,4,4,4-hexafluoro-2-butene according to any one of [8] to [11], in which the copper concentration is measured by X-ray photoelectron spectroscopy.
[13] The method for storing (E)-1,1,1,4,4,4-hexafluoro-2-butene according to any one of [8] to [12], in which the purity of the (E)-1,1,1,4,4,4-hexafluoro-2-butene to be filled is 99.90% by volume or more.

### Advantageous Effects of Invention

According to the present invention, the purity of the filled (E)-1,1,1,4,4,4-hexafluoro-2-butene is less likely to decrease over a long term.

### Description of Embodiments

One embodiment of the present invention will be described below. This embodiment describes one example of the present invention, and the present invention is not limited to this embodiment. Further, this embodiment can be variously altered or modified and embodiments obtained by such alternations or modifications may also be included in the present invention.

(E)-1,1,1,4,4,4-hexafluoro-2-butene is considered for utilization as a dry etching gas for manufacturing semiconductors, but is not widely used industrially. Therefore, the physical properties thereof have not been sufficiently elucidated, and there have been almost no reports on the long-term storage stability and compounds serving as decomposition catalysts.

As a result of intensive studies, the present inventors have found that (E)-1,1,1,4,4,4-hexafluoro-2-butene causes reactions, such as isomerization, polymerization, and decomposition, upon contact with metallic copper, copper alloys, or copper compounds, leading to a decrease in purity, and thus have completed the present invention. The present invention defines the material of a filled container filled with (E)-1,1,1,4,4,4-hexafluoro-2-butene to suppress the reactions, such as isomerization, polymerization, and decomposition, of (E)-1,1,1,4,4,4-hexafluoro-2-butene.

More specifically, a gas-filled container according to this embodiment is a filled container filled with (E)-1,1,1,4,4,4-hexafluoro-2-butene, in which a part in contact with the filled (E)-1,1,1,4,4,4-hexafluoro-2-butene of the filled container is formed of a metal having a copper concentration of less than 0.5% by mass.

A method for storing (E)-1,1,1,4,4,4-hexafluoro-2-butene according to this embodiment includes filling (E)-1,1,1,4,4,4-hexafluoro-2-butene into a container for storage, in which a part in contact with the filled (E)-1,1,1,4,4,4-hexafluoro-2-butene of the filled container is formed of a metal having a copper concentration of less than 0.5% by mass.

The part in contact with the filled (E)-1,1,1,4,4,4-hexafluoro-2-butene of the filled container is formed of a metal having a copper concentration of less than 0.5% by mass, and therefore the filled (E)-1,1,1,4,4,4-hexafluoro-2-butene is less likely to cause the reactions, such as isomerization, polymerization and decomposition. Therefore, even when the gas-filled container according to this embodiment is stored over a long term, the purity of the filled (E)-1,1,1,4,4,4-hexafluoro-2-butene is less likely to decrease. Accordingly, in the case of the gas-filled container filled with high-purity (E)-1,1,1,4,4,4-hexafluoro-2-butene, the high purity is likely to be maintained even after the long-term storage.

The copper refers to a copper element. When the metal forming the part in contact with the filled (E)-1,1,1,4,4,4-hexafluoro-2-butene of the filled container contains copper, the copper may be metallic copper, copper alloys, or copper compounds, such as copper salts.

The concentration of copper contained in the metal is required to be less than 0.5% by mass and preferably 0.4% by mass or less. To further suppress the reactions, such as isomerization, polymerization, and decomposition, of (E)-1,1,1,4,4,4-hexafluoro-2-butene, the concentration is more preferably 0.1% by mass or less and still more preferably 0.01% by mass or less. There is particular no lower limit for the copper concentration, but the concentration may be 0.001% by mass or more.

A method for measuring the copper concentration is not particularly limited, and the copper concentration can be measured by X-ray photoelectron spectroscopy.

### [Filled container]

The filled container in the gas-filled container according to this embodiment and the filled container in the method for storing (E)-1,1,1,4,4,4-hexafluoro-2-butene according to this embodiment preferably include a cylinder and a valve. The cylinder is a member containing (E)-1,1,1,4,4,4-hexafluoro-2-butene. The cylinder is preferably an integrally-molded seamless container.

The valve is a member opening and closing a flow passage for causing (E)-1,1,1,4,4,4-hexafluoro-2-butene inside the cylinder to flow to the outside and controlling the flow of (E)-1,1,1,4,4,4-hexafluoro-2-butene flowing through the flow passage.

The parts in contact with the filled (E)-1,1,1,4,4,4-hexafluoro-2-butene of the filled container, such as the cylinder (particularly the inner surface of the cylinder) and the valve, are preferably formed of steel. Steel includes at least one of stainless steel, manganese steel, and chromium molybdenum steel.

Cylinders formed of manganese steel and chromium molybdenum steel include, for example, cylinders manufactured from steel tubes specified in JIS standard JIS G3429 (seamless steel tubes for high-pressure gas cylinders), such as STH11, STH12 (manganese steel tubes) and STH21, STH22 (chromium molybdenum steel tubes).

However, these standards do not include items for copper, and thus the concentration of copper contained in manganese steel and chromium molybdenum steel is unknown, and therefore it is not enough to simply satisfy these standards. As a result of analyzing the inner surface of a plurality of cylinders actually on the market and satisfying the STH12 standard, some having a copper concentration of 1.0% by mass and some having a copper concentration of less than 0.005% by mass were mixed. Therefore, the use of common manganese steel and chromium molybdenum steel in the present invention requires the selection of those having a copper concentration of less than 0.5% by mass.

The valve is preferably formed of steel as with the cylinder. However, when plated with nickel plating or the like and prevented from directly contacting (E)-1,1,1,4,4,4-hexafluoro-2-butene, even valves formed of copper alloys, such as brass and Monel (registered trademark), are usable.

### [(E)-1,1,1,4,4,4-hexafluoro-2-butene]

The purity of (E)-1,1,1,4,4,4-hexafluoro-2-butene to be filled into the container (purity before being filled into the container) is preferably 99.90% by volume or more, more preferably 99.95% by volume or more, and still more preferably 99.99% by volume or more.

When the high-purity (E)-1,1,1,4,4,4-hexafluoro-2-butene described above is filled into the container, the purity hardly decreases during storage, and therefore the high purity is likely to be maintained even after the long-term storage.

For example, when the purity of (E)-1,1,1,4,4,4-hexafluoro-2-butene before being filled into the container is defined as X and the purity of (E)-1,1,1,4,4,4-hexafluoro-2-butene after being stored in the filled container at 23°C for 30 days is defined as Y, the purity Y can be set to 99.90% by volume or more.
Further, a difference between the purity X and the purity Y (purity X - purity Y) can be set to less than 0.02 percentage point.

When dry etching is performed using (E)-1,1,1,4,4,4-hexafluoro-2-butene having a purity of 99.90% by volume or more as a dry etching gas, the behavior of plasma and the reproducibility of the etching performance are likely to be improved.

A method for measuring the purity of (E)-1,1,1,4,4,4-hexafluoro-2-butene is not particularly limited, and the purity can be measured by gas chromatography or Fourier transform infrared spectroscopy (FT-IR spectroscopy).

A method for filling (E)-1,1,1,4,4,4-hexafluoro-2-butene into the container is not particularly limited. For example, a method is mentioned which includes filling (E)-1,1,1,4,4,4-hexafluoro-2-butene into an evacuated filled container through a filling line purged with an inert gas selected from a nitrogen gas (N₂), argon (Ar), and helium (He).

The purge may be either cycle purge in which the filling line is filled with an inert gas, and then evacuated or flow purge in which the inert gas is continuously caused to flow through the filling line.

The filling line is preferably constituted by a tube the inner surface of which is passivated or electropolished.

Examples of products generated by reactions resulting from the contact of (E)-1,1,1,4,4,4-hexafluoro-2-butene with copper include, for example, (Z)-1,1,1,4,4,4-hexafluoro-2-butene generated by cis-trans isomerization, 1,2,3,4-tetrakis (trifluoromethyl)cyclobutane, generated by dimerization, and polymers of (E)-1,1,1,4,4,4-hexafluoro-2-butene generated by polymerization.

### EXAMPLES

The present invention is more specifically described with reference to Examples and Comparative Examples below.

### [Example 1]

A 10 L capacity seamless manganese steel container having a copper concentration of 0.4% by mass was prepared as a cylinder. The inner surface of the cylinder was shot blasted, acid cleaned, and washed with water, and further dried. Thereafter, a SUS316L valve having a copper concentration of less than 0.005% by mass was attached to the cylinder, thereby obtaining a container. Then, the inside of the container was evacuated in a heated state.

The container was connected to a gas filling line connected to a SUS316 tank containing (E)-1,1,1,4,4,4-hexafluoro-2-butene. The purity X of (E)-1,1,1,4,4,4-hexafluoro-2-butene in the tank analyzed by gas chromatography is 99.95% by volume. The inner surface of this tank was electropolished.

Next, the gas filling line was subjected to the cycle purge of filling the gas filling line with a nitrogen gas, and then repeating evacuation, and then 1 kg of (E)-1,1,1,4,4,4-hexafluoro-2-butene was transferred from the tank to the container through the gas filling line, thereby obtaining a gas-filled container in which the filled container was filled with (E)-1,1,1,4,4,4-hexafluoro-2-butene. The internal pressure (gauge pressure) of the obtained gas-filled container was 0.06 MPaG.

The gas-filled container thus obtained was filled with (E)-1,1,1,4,4,4-hexafluoro-2-butene and allowed to stand still at 23°C for 30 days. Thereafter, the purity Y of (E)-1,1,1,4,4,4-hexafluoro-2-butene in the filled container was measured. As a result, the purity did not decrease from the purity X before being filled into the container and was 99.95% by volume. More specifically, the difference between the purity X and the purity Y (purity X - purity Y) was 0.00 percentage point. Table 1 shows the results.

**[Table 1]**

| | Cylinder | | Valve | | Purity X before filling (% by volume) | Purity Y after filling (% by volume) | Purity X - Purity Y |
|---|---|---|---|---|---|---|---|
| | Material | Copper concentration (% by mass) | Material | Copper concentration (% by mass) | | | |
| Ex. 1 | Manganese steel | 0.4 | SUS316L | Less than 0.005 | 99.95 | 99.95 | 0.00 |
| Ex. 2 | Manganese steel | 0.05 | SUS316L | Less than 0.005 | 99.95 | 99.95 | 0.00 |
| Ex. 3 | Manganese steel | Less than 0.005 | SUS316L | Less than 0.005 | 99.95 | 99.95 | 0.00 |
| Ex. 4 | Chromium molybdenum steel | 0.4 | SUS316L | Less than 0.005 | 99.95 | 99.95 | 0.00 |
| Ex. 5 | Chromium molybdenum steel | 0.05 | SUS316L | Less than 0.005 | 99.95 | 99.95 | 0.00 |
| Ex. 6 | Chromium molybdenum steel | Less than 0.005 | SUS316L | Less than 0.005 | 99.95 | 99.95 | 0.00 |
| Ex. 7 | Manganese steel + nickel alloy coating | Less than 0.005 | SUS316L | Less than 0.005 | 99.95 | 99.95 | 0.00 |
| Comp. Ex. 1 | Manganese steel | 0.5 | SUS316L | Less than 0.005 | 99.95 | 99.89 | 0.06 |
| Comp. Ex. 2 | Manganese steel | 1.0 | SUS316L | Less than 0.005 | 99.95 | 99.10 | 0.85 |
| Comp. Ex. 3 | Manganese steel | Less than 0.005 | Brass | 70 | 99.95 | 98.84 | 1.11 |
| Comp. Ex. 4 | Chromium molybdenum steel | 0.5 | SUS316L | Less than 0.005 | 99.95 | 99.88 | 0.07 |
| Comp. Ex. 5 | Chromium molybdenum steel | 1.0 | SUS316L | Less than 0.005 | 99.95 | 99.12 | 0.83 |
| Comp. Ex. 6 | Chromium molybdenum steel | Less than 0.005 | Brass | 70 | 99.95 | 98.89 | 1.06 |
| Comp. Ex. 7 | Manganese steel + nickel alloy coating | Less than 0.005 | Brass | 70 | 99.95 | 98.88 | 1.07 |

After the measurement of the purity Y of (E)-1,1,1,4,4,4-hexafluoro-2-butene, the filled container was subjected to the cycle purge of extracting (E)-1,1,1,4,4,4-hexafluoro-2-butene and charging a nitrogen gas, and then repeating evacuation. Then, the filled container was cut into 2 cm squares using a laser cutting machine, and then the inner surface of the filled container was subjected to XPS analysis using the resultants as measurement samples, thereby measuring the copper concentration. As a result, the copper concentration did not change from the initial 0.4% by mass.

An analyzer and analysis conditions used for the gas chromatography are as follows.
Analyzer: Gas Chromatograph GC-2014s manufactured by Shimadzu Corporation
Column: CarboPak B 60/80 SP-1000
Column temperature: 150°C/200°C
Injection temperature: 200°C
Carrier gas: Helium
Detector: Flame ionization detector (FID)

An analyzer, analysis conditions, and sputtering conditions used for the XPS analysis are as follows.
Analyzer: X-ray photoelectron spectrometer PHI5000VersaProbeII manufactured by ULVAC-Phi, Inc.
Atmosphere: Vacuum (less than 1.0 × 10⁶ Pa)
X-ray source: Monochromatic Al Ka (1486.6 eV)
Spectrometer: Electrostatic concentric hemispherical spectrometer
X-ray beam diameter: 100 um (25 W, 15 kV)
Signal capture angle: 45.0°
Pass energy: 23.5 eV
Measurement energy range:
   Cr2p 570-584 eV
   Mn2p 632-648 eV
   Fe2p 704-720 eV
   Cu2p 930-945 eV
Sputtering ion source: Ar2, 500+
Sputtering acceleration voltage: 10 kV
Sputtering region: 2 mm × 2 mm
Sputtering time: 10 minutes

### [Examples 2 to 6 and Comparative Examples 1, 2, 4, 5]

The purity Y of (E)-1,1,1,4,4,4-hexafluoro-2-butene in each gas-filled container after allowed to stand still at 23°C for 30 days was measured by performing the same operation as the operation in Example 1, except that steel forming the seamless container used as the cylinder (steel grade and copper concentration are shown in Table 1) was different. Table 1 shows the results.

### [Example 7]

The purity Y of (E)-1,1,1,4,4,4-hexafluoro-2-butene in a gas-filled container after allowed to stand still at 23°C for 30 days was measured by performing the same operation as the operation in Example 1, except that a nickel alloy coating was formed on the inner surface of a cylinder by an electroless plating method. Table 1 shows the results. The copper concentration of the surface of the nickel alloy coating was confirmed to be less than 0.05% by mass by XPS analysis.

### [Comparative Example 3]

The purity Y of (E)-1,1,1,4,4,4-hexafluoro-2-butene in a gas-filled container after allowed to stand still at 23°C for 30 days was measured by performing the same operation as the operation in Example 3, except for using a brass valve. Table 1 shows the results. The copper concentration of brass is 70% by mass.

### [Comparative Example 6]

The purity Y of (E)-1,1,1,4,4,4-hexafluoro-2-butene in a gas-filled container after allowed to stand still at 23°C for 30 days was measured by performing the same operation as the operation in Example 6, except for using a brass valve. Table 1 shows the results. The copper concentration of brass is 70% by mass.

### [Comparative Example 7]

The purity Y of (E)-1,1,1,4,4,4-hexafluoro-2-butene in a gas-filled container after allowed to stand still at 23°C for 30 days was measured by performing the same operation as the operation in Example 7, except for using a brass valve. Table 1 shows the results. The copper concentration of brass is 70% by mass.

As is understood from the results shown in Table 1, Examples 1 to 7 did not exhibit a decrease in purity due to the storage at 23°C for 30 days and had the difference between the purity X and the purity Y of 0.00 percentage point. In contrast thereto, a decrease in purity due to the storage at 23°C for 30 days was observed in Comparative Examples 1 to 7. It is considered in Comparative Examples 1 to 7 that the reactions, such as isomerization, polymerization, and decomposition, of (E)-1,1,1,4,4,4-hexafluoro-2-butene by copper occurred.

## Claims

1. A gas-filled container comprising:
a filled container filled with (E)-1,1,1,4,4,4-hexafluoro-2-butene, wherein
a part in contact with the filled (E)-1,1,1,4,4,4-hexafluoro-2-butene of the filled container is formed of a metal having a copper concentration of less than 0.5% by mass.

2. The gas-filled container according to claim 1, wherein the part in contact with the filled (E)-1,1,1,4,4,4-hexafluoro-2-butene of the filled container is formed of a metal having a copper concentration of less than 0.4% by mass.

3. The gas-filled container according to claim 1 or 2, wherein the metal is steel.

4. The gas-filled container according to claim 1 or 2, wherein the metal is at least one of manganese steel and chromium molybdenum steel.

5. The gas-filled container according to any one of claims 1 to 4, wherein the copper concentration is measured by X-ray photoelectron spectroscopy.

6. The gas-filled container according to any one of claims 1 to 5, wherein the filled container includes:
a cylinder containing the (E)-1,1,1,4,4,4-hexafluoro-2-butene; and
a valve configured to open and close a flow passage for causing the (E)-1,1,1,4,4,4-hexafluoro-2-butene inside the cylinder to flow to an outside.

7. The gas-filled container according to any one of claims 1 to 6, wherein a purity of the (E)-1,1,1,4,4,4-hexafluoro-2-butene to be filled is 99.90% by volume or more.

8. A method for storing (E)-1,1,1,4,4,4-hexafluoro-2-butene comprising:
filling (E)-1,1,1,4,4,4-hexafluoro-2-butene into a container for storage, wherein
a part in contact with the filled (E)-1,1,1,4,4,4-hexafluoro-2-butene of the filled container is formed of a metal having a copper concentration of less than 0.5% by mass.

9. The method for storing (E)-1,1,1,4,4,4-hexafluoro-2-butene according to claim 8, wherein the part in contact with the filled (E)-1,1,1,4,4,4-hexafluoro-2-butene of the filled container is formed of a metal having a copper concentration of less than 0.4% by mass.

10. The method for storing (E)-1,1,1,4,4,4-hexafluoro-2-butene according to claim 8 or 9, wherein the metal is steel.

11. The method for storing (E)-1,1,1,4,4,4-hexafluoro-2-butene according to claim 8 or 9, wherein the metal is at least one of manganese steel and chromium molybdenum steel.

12. The method for storing (E)-1,1,1,4,4,4-hexafluoro-2-butene according to any one of claims 8 to 11, wherein the copper concentration is measured by X-ray photoelectron spectroscopy.

13. The method for storing (E)-1,1,1,4,4,4-hexafluoro-2-butene according to any one of claims 8 to 12, wherein a purity of the (E)-1,1,1,4,4,4-hexafluoro-2-butene to be filled is 99.90% by volume or more.
